# EUROPÄISCHE PATENTSCHRIFT

(11) **EP 1 301 169 B1**
(45) Veröffentlichungstag und Bekanntmachung des Hinweises auf die Patenterteilung: **26.05.2004**
(21) Anmeldenummer: 01953186.2
(22) Anmeldetag: 29.06.2001
(51) Int. Cl.: A61K 7/48

(54) **EINEN SAUERSTOFFTRÄGER,AUSGEWÄHLT AUS HÄMOGLOBIN ODER HÄMOGLOBIN- UND MYOGLON-ENTHALTENDE ZUBEREITUNG IN FORM EINER EMULSION ALS KOSMETISCHES EXTERNUM UND ZUR NATÜRLICHEN REGENERATION DER HAUT BEI SAUERSTOFF-MANGEL**
PREPARATION IN THE FORM OF AN EMULSION THAT CONTAINS AN OXYGEN CARRIER SELECTED FROM HEMOGLOBIN OR HEMOGLOBIN AND MYOGLOBIN, FOR USE AS A TOPICALLY APPLICABLE COSMETIC AND FOR THE NATURAL REGENERATION OF THE SKIN IN THE CASE OF OXYGEN DEFICIENCY
SUPPORT D'OXYGENE, CHOISI PARMI L'HEMOGLOBINE OU UNE PREPARATION CONTENANT DE L'HEMOGLOBINE ET DE LA MYOGLOBINE, SOUS FORME D'UNE EMULSION COSMETIQUE POUR APPLICATION EXTERNE, POUR LA REGENERATION NATURELLE DE LA PEAU EN CAS DE MANQUE D'OXYGENE

(30) Priorität: 19.07.2000 DE 10034970
(43) Veröffentlichungstag der Anmeldung: 16.04.2003
(73) Patentinhaber: Sanguibiotech GmbH, 58455 Witten (DE)
(72) Erfinder: BARNIKOL, Wolfgang, 55128 Mainz (DE)
(74) Vertreter: Müller, Claudia Dr.
(86) Internationale Anmeldenummer: PCT/EP2001/007495
(87) Internationale Veröffentlichungsnummer: WO 2002/005754

(56) Entgegenhaltungen:
- FR-A- 2 616 659
- FR-A- 2 635 458
- FR-A- 2 641 463
- FR-A- 2 741 266
- DATABASE WPI Week 198411 Derwent Publications Ltd., London, GB; AN 1984-065027 XP002187417 & JP 59 020212 A (SHISEIDO), 1. Februar 1984 (1984-02-01)

## Beschreibung

Die Erfindung betrifft eine anspruchsgemäße Emulsion in Form einer Creme oder einer Lotion, die eine pflegerische Wirkung auf die Haut besitzt und zugleich die diffusive Sauerstoffversorgung der Epidermis von außen zu ihrer Regeneration und Behebung eines Sauerstoffmangels verstärkt. Das Mittel wird in die Haut eingerieben. Der Sauerstoff-Träger ist Hämoglobin allein oder ein Hämoglobin/Myoglobin-Gemisch .Er kann stabilisiert inaktiv oder aktiv und /oder modifiziert vorliegen . Überraschenderweise erfolgt eine verstärkte diffusive Sauerstoff-Versorgung der Haut von außen durch den Einsatz von einer oder mehreren Ölkomponenten zusammen mit einem oder mehreren O/W-Emulgatoren , ohne dass die emulsionsbildenden Komponenten die Stabilität des Sauerstoff-Trägers und dessen Diffusion beeinträchtigt werden.. Darüberhinaus kann mit der Zubereitung eine Farbgebung entsprechend der normalen gesunden Hautfarbe des Menschen erzielt werden, wobei im Gegensatz zu bisherigen derartigen Farbkosmetika ein natürlicher Farbstoff vorhanden ist. Die Emulsion führt darüberhinaus zu einer zusätzlichen Feuchtigkeitsversorgung der Haut.. Eine erfindungsgemäße Emulsion eignet sich daher als Kosmetikum und auch insbesondere bei degenerativen Hautveränderungen, wie beispielsweise nach Strahlung, Überhitzung (Verbrennung), bei Alterung, nicht nur zur Therapie, sondern auch zur Prävention, auch zusammen mit einer intravasalen Sauerstoff-Therapie. Die Erfindung betrifft ferner ein Verfahren zur Herstellung der charakterisierten Emulsion sowie deren Verwendung, insbesondere auch zusammen mit einem Sauerstoff-Träger- haltigen Gel, wobei der Sauerstoff-Träger dem o.g. entspricht.

Eine Reihe degenerativer Veränderungen der Haut werden durch chronischen Sauerstoffmangel hervorgerufen. Ein solcher Mangel entsteht in aller Regel dann, wenn die Haut nicht mehr ausreichend durchblutet ist. Dies geschieht entweder bei einer Einengung der kleinen Arterien - der Blut- Zufuhrgefäße - oder aber im Falle eines venösen Staus - Venen sind die Abflußgefäße des Organismus; betroffen sind vorzugsweise die Beine.
Der Kliniker kennt hierzu ganz bestimmte (dermatologische) Krankheitsbilder, beispielsweise die chronische periphere Verschlußkrankheit mit ihren vier verschiedenen Stadien nach FONTAINE oder die diabetische Angiopathie, deren Ursache eine Arteriosklerose ist, oder auch die chronische Veneninsuffizienz, d.h. eine Malfunktion von Gefäßen.
Der chronische Sauerstoffmangel führt schließlich zum geweblichen Zerfall der Haut, auch in Form der Gangrän oder des Ulcus cruris, sogen. offenes Bein, besonders häufig im Falle des Diabetes mellitus. Ist die Sauerstoffversorgung grenzwertig, was oft für alte Menschen zutrifft, so führen schon relativ kurzzeitige Kompressions-Anämien, wie sie während des festen Liegens vorkommen, oder nur geringfügiges Anstossen der Haut zu schnellem Zerfall zuerst der Haut und darauf auch zum Untergang der darunter liegenden Gewebe, was man als Dekubitus bezeichnet. Offensichtlich kommt es in diesem Fällen zur kutanen Narbenbildung. Ein wirksames Mittel muß demgemäß auch wirksam gegen solche Narben sein. Es wäre von Vorteil, hierdurch vorbeugende Methoden anwenden zu können, um prophylaktisch die genannten krankhaften und schmerzhaften Zustände zu vermeiden.
Eine ausführliche Darlegung der dermatologischen Klinik hierzu findet sich bei Braun-Falco, "Dermatologie und Venerologie", Springer-Verlag, lSBN 3-540-53542-X.

Weitere wichtige dermatologische Problemfelder sind Hautschäden nach Bestrahlungen. Man findet in diesem Fall entzündliche und degenerative Veränderungen. Es kann somit auch hier davon ausgegangen werden, dass eine verbesserte diffusive Sauerstoffversorgung der Haut von außen solche Schädigungen hintanhalten kann; möglich ist auch hierbei eine prophylaktische Therapie.
Ein drittes wichtiges Problemfeld sind Hautschäden nach Verbrennungen; auch hierbei könnte eine verstärkte diffusive Sauerstoffversorgung von außen helfen, die Haut besser und schneller zu regenerieren.

Die sichtbare äußere Schicht der Haut besteht aus etwa 15 Zelllagen verhomender, d.h. absterbender, sehr flacher Zellen (Hornzellen), diese Schicht (Stratum comeum) ist an der normalen Haut etwa 12 µm dick, was in etwa dem Durchmesser rundlicher Körperzellen entspricht. Die Hornzellen schilfern ständig ab und entstehen durch Teilung im darunter befindlichen sogen. Stratum germinativum. Es dauert etwa 1 Monat bis eine Basalzelle des Stratum germinativum an der Oberfläche als Keratinozyt von der Hautoberfläche abgestoßen wird. Die beiden Zellschichten der Epidermis (Stratum comeum und geminativum) sind zusammen etwa 30 µm dick. Damit bestehen für die Sauerstoffdiffusion, was die geometrischen Verhältnisse betrifft, innergewebliche Verhältnisse; denn der Versorgungsbereich einer Kapillare - das ist die kleinste Gefäßart des Organismus - besitzt eine Tiefe von rund 50 µm.

Dass - phylogenetisch gesehen - unsere Haut ein Gasaustausch-Organ war, zeigen vergleichend physiologische Betrachtungen: Der Regenwurm, etwa 7 mm dick, nimmt beispielsweise seinen Sauerstoff vollständig über die Haut auf, ebenso der unter Wasser überwinternde Frosch. Dass die menschliche Haut atmet, d. h. Sauerstoff aufnimmt und Kohlendioxid abgibt, zeigt sich allein schon daran, dass mit Hilfe epikutaner Elektroden sowohl ein Sauerstoffpartialdruck und ein Kohlendioxid - partialdruck bestimmbar ist.

Für die Entwicklung eines Mittels zur Verbesserung der Sauerstoff-Diffusivität durch die Epidermis ist vor allem die Struktur des dicht gepackten Stratum comeum zu beachten. Die interzelluläre Matrix dieses Stratums besteht aus einer parallel zur Oberfläche - angeordneten lamellären Lipoidschichten, wo sich sehr viele wässerige Schichten mit Lipoid-Doppelschichten abwechseln (vergl. W. Umbach, Kosmetik Thieme Verlag, ISBN 3-13-712602-9).

Insbesondere die wässerigen Schichten stellen einen großen Diffusionswiderstand für den Sauerstoffstrom von außen dar. Das anvisierte Mittel muß jedoch in beiden Schichtarten die Sauerstoffdiffusivität erhöhen. Emulsionstechnisch gesehen, stellt das Stratum comeum eine sogen. W/O-Form (W/O: Wasser in Öl) dar (vergl. H. Mollet, A. Grubenmann, Formulierungstechnik, Willey-VCH, D - Weinheim).

Unter der Epidermis liegt die Dermis: Diese wölbt sich in Form vieler Papillen in die Epidermis hinein, und in jeder Papille befindet sich eine versorgende Kapillare mit ihrem arteriellen und venösen Ende. Von dieser Kapillare diffundiert der Sauerstoff auswärts zur unteren vitalen Schicht der Epidermis, dem genannten Stratum germinativum. Die Epidermis erhält nun den notwendigen Sauerstoff aber nicht nur von innen, sondern - wie gezeigt wurde (beispielsweise Großmann et al., Adv. Physiol. Sci. 25 (1981): Oxygen Transport to Tissue. 319-320 oder L.R. Fitzgerald, Physiol. Rev. 37 (1957) 325-336) - sie versorgt sich zu etwa 50% von außen diffusiv mit Sauerstoff.
Grundsätzlich sind die Bedingungen für die Diffusion des Sauerstoffes von außen durch die Epidermis günstiger als im Fall der kapillaren Sauerstoffabgabe. Denn anders als hier beträgt der Sauerstoffpartialdruck, als treibende Kraft der Diffusion, in der Luft 150 und nicht nur 50 mm Hg; zudem liegt in der Haut eine lineare Diffusionsgeometrie vor und keine zentrifugale.
Anders als im Fall des im Organismus überwiegenden Sauerstofftransports via Lunge als diffusives Sauerstoffaufnahme-Organ, via den konvektiven vaskulären Transport und via Diffusion von der Kapillare zu den Zellen, sind Aufnahme und Abgabe des Sauerstoffes in der Haut räumlich dicht beieinander; es gibt hier keinen konvektiven Sauerstofftransport, die beiden diffusiven Vorgänge - Aufnahme und Abgabe - sind miteinander verschmolzen.
Dennoch bleiben die grundlegenden Betrachtungen der Sauerstoffaufnahme und - abgabe erhalten: Der Sauerstoff muß aus der Luft mit ausreichender Affinität gebunden und andererseits, auch bei geringer Sättigung des Hämoglobins, mit möglichst hohem Sauerstoffpartialdruck diffusiv hin zu den vitalen Zellen des Stratum geminativum getrieben werden. Dies leistet die S-förmige Sauerstoffbindung des Hämoglobin bei einer geeignet eingestellten mittleren Affinität (i. e. Sauerstoffpartialdruck bei Halbsättigung: p50-Wert); ein Maß für die S-Förmigkeit der Bindung ist der Hillsche Index(n50-Wert). Die genannten Parameter der Sauerstoffbindung (p50 und n50) sollten für die Hautwirkung optimal eingestellt werden, derart, daß einerseits der Luftsauerstoff vollständig vom Hämoglobin gebunden wird, aber andererseits auch wieder in ausreichendem Maße an die Zellen des Stratum geminativum abgegeben wird.

Wie erläutert, besitzt die Matrix des Stratum corneum einen geschichteten Aufbau parallel zur Oberfläche und entspricht insgesamt einer sogenannten W/O-Emulsion. D. h., es wechseln molekulare hydrophile und lipophile Schichten einander ab. Der Sauerstoff-Diffusionswiderstand dieser Matrix muß also für die externe Sauerstoff-Versorgung verringert werden. Dazu muß der Diffusionswiderstand beider Schichtarten verkleinert werden, nämlich derjenige der hydrophilen und der lipophilen.

Bisher sind fetthaltige Emulsionen als einerseits kosmetische Produkte und andererseits als externe Sauerstoffversorger nicht bekannt, bei denen der Sauerstoff mittels Hämoglobin oder analogen natürlichen Stoffen transportiert wird, da diese Sauerstoffträger empfindlich, insbesondere gegenüber Emulgatoren,Fetten empfindlich reagieren können und ein Diffusionswiderstand, auch durch Fett-Emulgatorengemische bedingt, vorliegt.

Eine Gruppe von Patenten deckt gewisse therapeutische Maßnahmen ab, die die Regeneration der Haut betreffen. So lehrt EP 275109 A2 die Verwendung von Undulin, einem Glykoprotein, im Gemisch mit Prokollagen und Kollagen Typ1 zur Verhinderung der Hautalterung. Das gleiche Ziel wird in DE 19521828 A1 durch Anwendung von Kollagenase verfolgt. WO 99/26589 A2 lehrt die Verwendung von Chlorophyll, Kabeljau-Öl und Campher als aktive Bestandteile einer pharmazeutischen Bereitung zur Behandlung von Hautverbrennungen, Sonnenbrand, Hautverbrühungen, Hautirritation und Hautabschürfungen.

Bezüglich Farbeffekten auf der Haut lehrt EP 656920 B1 die Verwendung eines Benzopyran-Derivates. WO 99/11718 A1 betrifft ein stabiles Extemum mit sehr vielen natürlichen pfanzlichen Farbstoffen; Hämoglobin wird nicht genannt. DE 4200349 C2 lehrt die Verwendung von Chlorophyll als natürlichen Farbstoff, welches an Apohämoglobin zwecks Stabilisierung gebunden wird.
EP 706788 B1 behandelt saure Farbstoffe, die das ergraute Haar in seine ursprüngliche Farbe zurückfärbt.
Schließlich lehrt EP 992236 A1, wie stark pigmentierte Haut wieder aufgehellt werden kann und zwar durch Anwendung von Phospholipid, Antioxidantien, bestimmter Proteine und bestimmter Mukopolysaccharide.

EP-B 0 673 643 beschreibt kosmetische Zusammensetzungen, welche eine Kombination aus Superoxid-Dismutase (SOD) und einem Parphyrin wie Chlorophyll oder Hämoglobin enthalten.

DE 42 36 607 betrifft Zubereitungen zur verbesserten Abgabe von Sauerstoff in die Haut mit Fluorcarbonen als wesentliche Komponente.

DE-PS 39 90 820 (C2) offenbart Lichtschutzmittel für Hautzellen auf Basis von Ribonucleinsäuren.

JP 05 310 597 A betrifft ein Mittel zur Kontaktbehandlung der Haut zur Schmerzlinderung, enthaltend eine Elektronen-akzeptierende Verbindung, wobei als solche z.B. Chlorophyll oder Hämoglobin eingesetzt werden kann, welche in eine elektrisch leitende Matrix eingebaut wird.

JP 02019311 A betrifft Sonnenschutzmittel, enthaltend Hämin oder Hämatin, in Porphyrinverbindungen.

JP 62111907 A beschreibt Globin-enthaltende Feuchtigkeits-, Sonnenschutz- und Erythem-hemmende Mittel, wobei Blutkomponenten im Globin interpariert werden.

Lipoide Emulsionen werden für diese Mittel nicht beschrieben.

Aufgabe vorliegender Erfindung ist daher die Entwicklung eines Mittels zur Steigerung der externen Sauerstoff-Versorgung der Haut, mit der die oben genannten Sauerstoffmangel-Erkrankungen behandelt und verhindert werden können, wobei gleichzeitg die Haut insbesondere gepflegt wird durch ausreichende Versorgung mit Feuchtigkeit, ferner der Sauerstoff-Träger nicht degeneriert und zusätzlich eine Färbung der Haut gemäß der biologisch ursprünglichen Hautfarbe möglich ist. Ferner soll eine leichte Anwendung, durch Eintragen in die Haut erfolgen.

Diese Aufgabe wird nun erfindungsgemäß dadurch gelöst, daß in eine geeignete Emulsion 0,1-30% Hämoglobin oder Hämoglobin und 0,1-50 % Myoglobin, bezogen auf die Hämoglobin - Menge der Sauerstoffträger, eingearbeitet werden.
Die Emulsion ist erfindungsgemäß eine Öl-in-Wasser-Emulsion.
Überraschenderweise zeigte sich, dass mit einer derartigen Emulsion ein Eindringen des Sauerstoffs in die Epidermis möglich ist. Die eingesetzten nachfolgend beschriebenen Ölkomponenten und die Emulgatoren bewirken keine Destabilisierung des Sauerstoff-Trägers und führen umgekehrt zu einer Feuchtigkeitsversorgung ( Pflegeeffekt ) der Haut bei gleichzeitiger natürlicher Farbgebung. Darüberhinaus ist überraschenderweise die Sauerstoff-Diffusion nicht behindert.

Überraschenderweise zeigte sich, dass der Diffusionswiderstand der lipophilen Schichten der Haut mit den nachfolgend genannten Ölkomponenten, verringert werden kann. Erfindungsgemäß führen diese für Sauerstoff zu einem erhöhten BUNSENschen -Lösichkeitshoeffizienten (∝) und verkleinern somit - dem ersten FICKschen Gesetz gemäß und weil stets eine Partialdruckdifferenz des Sauerstoffs die treibende Kraft für die Diffusion darstellt - den Diffusionswiderstand der lipophilen Schichten der Haut. Dies führt zu einer guten Wirksamkeit der Emulsion, da diese Öle sich nach dem Einarbeiten der Emulsion in die lipophilen Schichten der Haut hineinlösen und somit diese Schichten strukturell auflockern. Obwohl diese Auflockerung generell durch die Dickezunahme zu einem erhöhten Widerstand (P. Vaupel, Pflügers Archiv 361 (1976) 201 - 204) führt, ist erfindungsgemäß der Diffusionswiderstand insgesamt so gering, dass der Sauerstoff-Träger durch die Schichten der Epidermis eindringen und die Hautschichten mit Sauerstoff versorgen kann.

Die erfindungsgemäße Emulsion enthält vorzugsweise als Ölkomponente solche, die gemäß obiger Darlegung auch der Steigerung des Löslichkeitshoeffizienten des Sauerstoffs dienen, und ausgewählt sind aus insbesondere (Tri-)Glycerinestern mit C4-C22- ungesättigten oder gesättigten Fettsäuren wie Butter-,Valerian-,Capron-Önath-Capryl-,Pelargon-,Caprin-,Undecan-,Laurin-,Tridecan-,Myristin-,Pentadecan-Palmitin-, Margarin-Stearin-, Nonadedcan-, Arachin-Behensäure. In vielen insbesondere natürlichen Fetten liegen meist Gemische einzelner Fettsäuren in den Glycerinestern vor,wie z.B. von Palmitin-,Stearin- und Ölsäure in tierischen Fetten. Ferner sind die verzweigten Produkte hiervon und/oder die ungesättigten geeignet wie Eruca, Sorbin-, Linol-, Linolen-, Elaeostearin, Arachidon, Clupanodon- oder Docosahexansäure-Fette. Insbesondere geeignet sind hieraus die Fette aus kurz (C4 bis C8)-kettigen und mittelkettigen (C8 bis C12, insbesondere bis C10) gesättigten unverzweigten oder verzweigten Fettsäuren wie Caprylsäure und Caprinsäure, Isobutter-und Valeriansäure.sowie Mischungen hiervon und Mischungen aus den kurzkettigen C4 bis C8 und/oder den mittelkettigen C8 bis C12, besonders bis C10-Fettsäure-Fetten.
Ferner sind erfindungsgemäß auch Öle mit ungesättigtem Säureteil wie Öl aus Soja, Aloe, Aprikose, Pflaume, Macademia, Rosen, Arnika, Avocado, Rizinus, Kümmel, Senf, Seam, Shea, Sonnenblume, Traubenkern, Nuß und Weizenkeim . geeignet. Die Öle können allein oder in Mischung oder in Mischung mit den Fetten der genannten Fettsäuren vorliegen. Insbesondere bevorzugt sind Fette aus den genannten kurzkettigen oder mittelkettigen Fettsäuren oder Mischungen hiervon insbesondere betreffend verzweigte und unverzweigte gesättigte, (ggf. ungesättigte), und ganz besonders erwünscht ist eine Mischung von o.g. Ölen und Fetten aus kurzkettigen C4-C8- und/oder mittelkettigen C8 bis C12, insbesondere bis C10-Fettsäure-Fetten oder deren Mischungen wie beschrieben. Ganz besonders bevorzugt ist eine solche Mischung im Verhältnis 1:1.

Als Emulgatoren kommen alle Typen von Emulsionsbildnern gemäß "Apothekenrezeptur und -defektur" (Karl Thoma, Deutscher Apothekerverlag, Stuttgart) in Frage. Ein Beispiel ist Unguentum emulsificans aquosum (vergl. DAB '97). Ferner kommen als cremophore Emulgatoren insbesondere solche für Feuchtigkeitsemulsionen in Frage, nämlich anonische, kationische, amphotere und nichtionische mit ihren charakteristischen HLB-Werten , nämlich 8-18 (HLB: Hydrophile.Lipophile-Balance), bevorzugt 8-15, insbesondere 9-13, insbesondere jedoch nichtionische Emulgatoren, wie Stearate oder Oleate oder Laurate mit Glycerol oder Glykol oder Polyethylenglykol oder Sorbit, wie auch z.B.polyoxyethylierte, und unter der Bezeichnung "Macrogof", "Tween", "Myrj" bzw. "Cremophor" bekannte Emulgatoren mit dem o.g. HLB-Wert (z.B. Macrogol-9-stearat , Sorbitanmonostearat), oder Mischungen von den genannten Emulgatoren, vergleiche auch K. Schrader, Grundlagen und Rezepturen der Kosmetika, 2. Auflage, Huthig-Verlag, Heidelberg, 1989. ISBN 3-7785-1491-1, Seiten 395-398. Beispielhaft sind in der nachfolgenden Tabelle entsprechend der Literaturstelle Schrader einige Emulgatoren, die erfindungsgemäß eingesetzt werden können, genannt:

| Handelsname | Chem. Bezeichnung | nichtionogen anionaktiv kationaktiv | HLB |
|---|---|---|---|
| Emulgator 157 | Propylenglykolmonostearat, selbstemulgierend | a | 7,5 |
| G-2147 | Tetraethylenglykolmonostearat | n | 7,7 |
| G-1425 | Polyethylenglykolsorbit-Lanolinderivat | n | 8,0 |
| Acacia - | USP | n | 8,0 |
| G-3608 | Polyoxypropylenstearat | n | 8,0 |
| Span 20 | Sorbitanmonolaurat | n | 8,6 |
| Arlacel 20 | Sorbitanmonolaurat | n | 8,6 |
| G-2111 | Polyoxyethylenoxypropylen oleat | n | 9,0 |
| G-2125 | Tetraethylenglykolmonolaurat | n | 9,4 |
| Brij 30 1 | Polyoxyethylenlaurylether | n | 9,5 |
| Tween 61 1 | Polyoxyethylensorbitanmonostearat | n | 9,6 |
| Gelatine | Pharmagel B' | n | 9,8 |
| Tween 81 | Polyoxyethylensorbitanmonooleat | n | 10,0 |
| Arlypon OAG | Fettalkoholpolyglykolether | n | 10,0 |
| G-3806 | Polyoxyethylencetylether | n | 10,3 |
| Tween 65 | Polyoxyethylensorbitantristearat | n | 10,5 |
| Methylcellulose - | Methocel 15 cps | | 10,5 |
| Lamecreme SA 7 | Fettalkoholglykolether | n | 10,9 |
| Tween 85 | Polyoxyethylensorbitantrioleat | n | 11,0 |
| G-1790 | Polyoxyethylenlanolinderivat | n | 11,0 |
| Myrj 45 | Polyethylenglykolmonostearat | n | 11,2 |
| Arlypon 0A8 | Polyoxyethylen-oleylalkoholether | | 11,3 |
| | Polyethylenglykol-400-monooleat | n | 11,4 |
| Cremophor S9 | Polyethylenglykol-400 monostearat | n | 11,6 |
| G-2161 | Polyethylenglykol-400 monostearat | n | 11,6 |
| Atlox 3300 | Alkylarylsulfonat | n | 11,7 |
| Lamecreme LPM | Glycerinmonodistearat | a | 12,0 |
| Atolx 3300 | Triethanolaminoleat | a | 12,0 |
| G-3910 | Polyoxyethylenoleylether | n | 12,2 |
| G-2127 | Polyoxyethylenmonolaurat | n | 12,8 |
| Renex 690 | Polyoxyethylenalkylarylether | n | 13,0 |
| Lamecrerne AOM | Glycerinmonodistearat | n | 13,0 |
| Lamecreme CSM | Glycerinmonodistearat | a | 13,0 |
| Lamecreme ZEM | Glycerinmonedistearat | a | 13,0 |
| Renex 690 | Polyethylenglykol-400-monooleat | n | 13,1 |
| Tragant USP | | n | 13,2 |
| Cremophor EL | Polyoxyethylen-Ricinusöl | n | 13,3 |
| G-1284 | Polyoxyethylen-Ricinusöl | n | 13,3 |
| Tween 21 | Polyoxyethylensorbitan monolaurat | n | 13,3 |
| Renex 20 | Polyoxyethylenester gemischter Fett- und Harzsäuren | n | 13,5 |
| Lamecreme KSM | Glycerinmonodistearat | a | 14,0 |
| G-1441 | Polyoxyethylensorbit Lanolinderivat | n | 14,0 |
| Lamacit 877 | Polyoxyethylen Alkylphenolether | | 14,7 |
| G-7596 J | Polyoxyethyl ensorbitan monolaurat | n | 14,9 |
| Lamacit CA | Polyoxyethylen-Fettalkohol ether | | 14,9 |
| Lamacit GML-12 | Polyoxyethylenglycerin monolaurat | n | 15,0 |
| Tween 60 | Polyoxyethylensorbitan monooleat | n | 15,0 |
| Myrj 49 | Polyoxyethylenmonostearat | n | 15,0 |
| Cremophor 0 | Polyoxyethylen-Fettalkoholether | n | 16,0 |
| G-1471 | Polyoxyethylensorbit-Lanolinderivat | n | 16,0 |
| Cetomacro gol-1000 | Polyethylenglykol- 100 monocetylether | n | 16,1 |
| Lamacit GMO-25 | Polyoxyethylenglycerinmonooleat | n | 16,2 |
| Lamacit GML-20 | Polyoxyethylenglycerinmonolaurat | n | 15,0 |
| Tween 20 | Polyoxyethylensorbitanmonolaurat | n | 16,7 |
| Brij 35 | Polyoxyethylenlaurylether | n | 16,9 |
| Emulgator GL-120 | Pentaerythrit-Lanolinpoly glykolether | n | 17,0 |
| ...... | Natriumoleat | a | 18,0 |

Die Emulsion enthält ferner Konservierungsmittel. Als Konservierungsstoffe sind beispielsweise Substanzen, wie Dibromhexamidin, 4-Dihydracetatsäure, 4-Hydroxybenzoesäure, Propionsäure, Salicylsäure, Sorbinsäure, Formaldehyd Paraformaldehyd, o-Phenol, anorganische Sulfite und Bisulfite, Natriumjodat, Chlorobutanol ,Citronensäure und Ameisensäure oder Mischungen hiervon geeignet, im Falle der Säuren kommen auch deren Esther und Salze in Frage, insbesondere Kalium-Sorbat.

Als Feuchthaltemittel können Substanzen wie Na-Laktat, Polylenglykol, Sobitol , Glycerin oder Pyrrolidoncarbonsäure oder Mischungen hiervon dienen.

Bevorzugt werden, aus Konservierungsmitteln ausgewählt, Methyl-4-Hydroxy-Benzoat und Propyl-4-Hydroxy-Benzoat, z.B. 0,02-0,25%, insbesondere 0,05-0,2%. Bevorzugte Feuchthaltemittel sind wie Na-Lactat, Glycerol, Propylenglycol, Propandiol, Sorbit, PCA (Pyrrolidoncarbonsäure), in einer Menge von 5-15% enthalten sein. Besonders bevorzugt sind Methyl-4-Hydroxy-Benzoat und Propyl-4-Hydroxy-Benzoat und Glycerol, Propylenglycol und Sorbit, insbesondere jeweils Mischungen hiervon, z.B. 1:1 betreffend Konservierungsmittel und 1:1:1 bezüglich Feuchthaltemittel (Prozente sind Massenanteile).

Mit der erfindungsgemäßen Zubereitung wird der Diffusionswiderstand der hydrophilen Schichten für den Sauerstoff durch die Gegenwart des eingearbeiteten Hämoglobins oder Hämoglobin/Myoglobin-Gemisches herabgesetzt über den Mechanismus der erleichterten Diffusion. Es entsteht eine sogenannte erhöhte Scheinlöslichkeit des Sauerstoffs.

Als fakultative Stoffe können die folgenden Komponenten oder Mischungen hiervon inkorporiert werden, welche ausgewählt sind aus Durchblutungsmitteln, Parfümstoffen, Substanzen zur Narbentherapie/Regeneration, Antioxidantien und antiinfektiösen Substanzen:

Für Substanzen zur Durchblutungsförderung gibt es zwei Möglichkeiten:
Erstens eignen sich hierzu bestimmte ätherische Öle, welche sich dann in der Lipoidphase der Emulsion befinden. Hierzu gehören Extrakte von Amika, Bay, Cassia, Kampfer, Limette, Majoran, Moschus, Narde, Nelke, Rosmarin, Thymian, Wacholder, Zimt, Ingwer, Weihrauch, roter und schwarzer Pfeffer, Eukalyptus. Die Gewichtsanteile der Substanzen in der Emulsion können 0 bis 13 % der Emulsion ausmachen, insbesondere jedoch zwischen 2 und 10 Gewichts%.
Zweitens kommen sogen. stark hyperämisierende, wie Nikotinamid, Nonivamid oder Kapsaicin, als durchblutungsfördernde Substanzen in Frage. Ihr Gewichtsanteil an der Emulsion kann zwischen 0 und 3, insbesondere jedoch zwischen 0 und 1,5 Gewichts% betragen.

Substanzen aus jeder oder den beiden o.g. Gruppen können auch kombiniert werden wie z.B.Nikotin und Rosmarin, wobei die Gesamtmenge dann 13% maximal beträgt.

Weiterhin kann die Emulsion auch anti-infektiöse Substanzen enthalten (antibakterielle, antivirale und antimykotische). Vorgesehen sind wiederum bestimmte ätherische Öle, wie geeignete Extrakte aus Ajowan, Jasmin, Kamille, Latschenkiefer, Lavendel, Magnolie, Muskatnuss, Ravensara, Sassafras, Thymian, Vetiver, Wacholder, Weinrebe, Zitrone, Tolubalsam, Pfefferminze, Myrte, Origano, Pinie, Senf, Zwiebel oder Mischungen hiervon.
Der Anteil an der Emulsion kann zwischen 0 und 13, insbesondere zwischen 2 und 10 Gewichts% betragen.

Ferner kann die Emulsion Substanzen, hauptsächlich wiederum ätherische Öle zur Narbenbehandlung und Regeneration enthalten. Solche sind beispielsweise aus Angelika, Geranie, Salbei, Rosmarin, Schafgarbe, Thuja, Juniperus virginiana, Zwiebel und Cedrus atlantica, oder Mischungen hiervon.
Der Gewichtsanteil solcher Substanzen kann zwischen 0 und 13 % betragen, insbesondere jedoch zwischen 2 und 10%.

Schließlich kann die Emulsion gegen eine Autoxidation mit sogenannten Antioxidantien geschützt werden, insbesondere, wenn sie ungesättigte Fettsäuren enthält. Solche Substanzen sind α-Tocopherol, Ascorbyl-Palmitat, tertiäres Butylhydroxy-Toluol (BHT), Butylhydrochinon und Butylhydroxy-Anisol oder Mischungen hiervon, und zwar zwischen 0,0001 und 0,5, bevorzugt zwischen 0,01 und 0,08 Gew%.

Viele der genannten Substanzen sind auch Parfümstoffe, wie z.B.Rosmarin, Salbei, Jasmin, Lavendel, Nelke. Als Parfümkomponenten können auch andere hierfür bekannte Substanzen Verwendung finden, wie z.B. Moschus, Neroli, Geranie, Mandarine, oder Mischungen hiervon.

Die genannten fakultativen Adjuvantien der Emulsion können einzeln oder kombiniert in der Emulsion enthalten sein; dies betrifft auch die einzelnen Substanzen innerhalb der genannten Gruppen. Die fakultativen Substanzen sind dann insgesamt in einer Menge von 0-20% vorhanden.

Besonders bevorzugt werden Narbenbehandlungsmittel, Durchblutungsmittel und antiinfektiöse Mittel, insbesondere im Verhältnis 1:1:1 miteinander kombiniert und liegen dann insgesamt in einer Menge von 2-20, insbesondere 5-13% vor. Wie erwähnt werden hierzu auch bevorzugt noch Antioxidantien,wenn erforderlich in der angegebenen Menge zugesetzt.

Generell enthält eine geeignete Emulsion zwischen 20 und 90 % Wasser, 10 - 80 % , insbesondere 20-60% Ölkomponente, 2 - 20 % , insbesondere 2-10 %, ganz besonders 2-5% oder 3-4,5% Emulgator, 5 -15% , insbesondere 8-12% Feuchthaltemittel und 0,02 - 0,25 , insbesondere 0,15-0,2% Konservierungsmittel.und 0-20% einer oder mehrerer der o.g. fakultativen Zusatzstoffe sowie 0,1-30% Hämogobin oder Hämoglobin/Myoglobin, wobei Myoglobin in Mengen von 0,1-50%, bezogen auf Hämoglobin vorliegen kann.

Die Angaben sind jeweils Gewichtsprozent.

Bei gleicher qualitativer Zusammensetzung hat die Creme stets einen geringeren Wassergehalt als die Lotion.
Ein Wassergehalt in der Emulsion, insbesondere 40 -80 Gew.%Wasser und 5-50Gew.% Öl ,ergibt eine Creme, ein Wassergehalt , insbesondere 70-90Gew.% Wasser und 1-20Gew.% Öl,führt zu einer fließfähigen Lotion.
Die Bereiche der Wassergehalte (Creme oder Lotion) hängen auch insbesondere vom verwendeten Emulgator ab.

Eine besonders bevorzugte Zubereitung umfaßt Hämoglobin oder Hämoglobin/Myoglobin ( 0,1-30%, insbesondere 0,1-20%, bezogen auf die Gesamtmasse), 5-15 % Feuchthaltemittel, 0,02-0,25 % Konservierungsmittel sowie 5-15 % Zusatzstoffe, ausgewählt aus allen Arten der Zusatzstoffe.

Ganz besonders bevorzugt sind Zubereitungen mit 0,02-0,08 Propyl-4-Hydroxybenzoat, sowie 0,07-0,15 Masseteile Methyl-4-Hydroxybenzoat, 8-12% Glycerol, Propylenglycol und/oder Sorbit (1:1 oder 1:1:1).sowie den angegebenen Mengen an Sauerstoff-Träger und Öl, Emulgator und Zusatzstoffen.
Insbesondere bevorzugt liegt Hämoglobin oder Hämoglobin und Myoglobin in der Emulsion in einer Menge von 2-8% vor. Die angegebenen Mengen sind jeweils Gewichtsprozente.

Die erfindungsgemäße Emulsion wird hergestellt, indem Wasser, Ölkomponente(n) und Emulgator(en) unter Rühren insbesondere bei Raumtemperatur,wie z.B.22°C zusammenfügt,ggf bei festen Bestandteilen erwärmt, z.B.auf 50 , insbesondere 60 bis zu 80°C und sodann ,insbesondere nach dem Abkühlen auf Raumtemperatur,die Zusatzstoffe, sofern vorhanden, zusetzt. Schließlich wird die getrennt hergestellte Hämoglobin -oder Hämoglobin/Myoglobin-Lösung hinzugefügt (bevorzugt bei Raumtemperatur).

Das Hämoglobin kann insbesondere bevorzugt in der erfindungsgemässen Zubereitung ein, ganz besonders bevorzugt mit Kohlenmonoxid (CO) stabilisiertes, Hämoglobin vom Menschen oder Rind, vorzugsweise jedoch Schweinehämoglobin sein. Die Herstellung eines solchen stabilisierten Hämoglobins ist in der DE 1 970 103.7 beschrieben (entsprechend US-Patent Nr. 5,985,332). Demnach kann Hämoglobin/Myoglobin durch Aquilibrierung mit CO vollständig in Carboxyhämoglobin/Myoglobin überführt werden, welches lagerstabil ist und vor der weiteren Anwendung nicht deligandisiert werden muss. Die Karbonylierung ist auch mit modifiziertem Hämoglobin möglich.

Die Aktivierung des Sauerstoffträgers erfolgt dann durch lokale Begasung der Haut mit Sauerstoff, auf die die Emulsion aufgetragen worden ist.

Das Hämoglobin kann, wie erwähnt, in einer Mischung mit Myoglobin, insbesondere letzteres in Mengen von 0,1 bis 50 %, bezogen auf die Hämoglobin-Menge, vorliegen. Bevorzugt wird Myoglobin mit Hämoglobin in Mengen von 50 bis 70% Hämoglobin und 50-30% Myoglobin, insbesondere 75 bis 90% Hämoglobin und 25 bis 10% Myoglobin eingesetzt. Die %-Angaben sind hierbei Masseanteile.

Das Hämoglobin oder Hämoglobin und Myoglobin liegen in der Hydrophase der Emulsion in bestimmten Konzentrationen, wie angegeben, in den erfindungsgemäß beschriebenen molekularen Formen, molekular-dispers vor.

Als Hämoglobin kann insbesondere Humanhämoglobin, Schweinehämoglobin oder Rinderhämoglobin eingesetzt werden. Die Art des Myoglobins ist ebenfalls wählbar, es kann von verschiedenen Tierarten gewonnen werden, wie z. B. vom Hund, vom Schaf, vom Pferd oder vom Wal.
Die oben genannten Hämoglobine/Myoglobine sind als solche bekannt und beispielsweise in "Prinzipien der Biochemie" von Lehninger, Nelson, Cox (Spectrum-Verlag) beschrieben.

Als geeignete Salze, welche bei der Bereitstellung des Sauerstoff-Trägers vorliegen können, natürliche Elektrolytkomponenten der Lösung des Hämoglobins/Myoglobins wie NaCl, KCI und NaHCO₃, insbesondere in folgenden physiologischen Mengen vorliegen (in mM): NaCl 125; KCl 4,5; NaHCO₃20.

Vorteilhafterweise werden native Hämoglobine eingesetzt.
Die eingesetzten Hämoglobine und Myoglobine können aber auch zur Stabilisation und verbesserten Verträglichkeit alternativ insbesondere mit Polyalkylenoxiden kovalent verknüpft sein, wie in US 4 179 337, US 5 478 805, US 5 386 014, EP 0 206 448, EP 0 67 029 beschrieben. Dies dient der Gewebeverträglichkeit sowie der Stabilisation der Produkte.
Kovalente Verknüpfungen von Polyalkylenoxiden an Proteine, insbesondere auch an (unvernetztes) Hämoglobin, sind etliche bekannt und in der Literatur beschrieben (den Stand der Technik beschreibt umfassend: Harris J. M. (Hrsg.): ***Poly (Ethylene*** ***Glycol) Chemistry: Biotechnical and Biomedical Applications,*** Plenum, New York u. a. 1992). Bei sehr vielen dieser Verfahren erfolgt die Anknüpfung des Polyalkylenoxides über eine molekulare Brücke *("spacer"),* die beispielsweise in bifunktioneller Verknüpfer schafft. Streng betrachtet wird in diesen Fällen also ein Verknüpfungsprodukt eines Polyalkylenoxids mit einem Verknüpfungsreagenz an das Protein geknüpft.

Zur kovalenten Anknüpfung der Polyalkylenoxide (Polyalkylenglykole) werden bevorzugt solche Derivate der Polyalkylenoxide verwendet, die ein verknüpfendes Agens mit einer funktionellen Gruppe bereits kovalent gebunden enthalten, welche eine direkte chemische Reaktion mit Amino-, Alkohol-, oder Sulfhydryl-Gruppen der Hämoglobine unter Bildung kovalenter Anknüpfungen der Polyalkylenoxide ergeben - beispielsweise Polyalkylenoxide mit reaktiven N-Hydroxysuccinimidylester-, Epoxid- (Glycidylether-), Aldehyd-, isocyanat-, Vinylsulfon-, Jodazetamid-, Imidazolylformat-, Tresylatgruppen, u. a. Viele solche monofunktionell aktivierte Polyethylenoxide sind kommerziell erhältlich. Alternativ können nicht-aktive Polyalkylenoxide in jeder weiteren geeigneten Weise zunächst chemisch aktiviert oder, eventuell nach einer zusätzlich notwendigen Derivatisierung, durch chemische Verknüpfungsagenzien mit Bromcyan, einem Karboiimid wie beispielsweise 1-Ethyl-3-(3-Dimethylaminopropyl)karbodiimid oder N,N'-Dizyklohexylkarboiimid, Cyanurchlorid (mit diesem aktivierte Polyethylenglykole, 4,6-Dichlor-s-triazin-Polyethylenglykole, sind ebenfalls kommerziell erhältlich), oder anderen, bekannten Verknüpfungsagenzien wie beispielsweise 2,2'-Dichlorbenzidin, p,p'-Difluor-m,m'dinitrodiphenylsulfon, 2,4-Dichlomitrobenzol und weiteren (Überblick in: Harris J. M. (Hrsg.): ***Poly (Ethylene Glycol) Chemistry: Biotechnical and Biomedical Applications,*** Plenum, New York u. a. 1992).
Als Polyalkylenoxide eignen sich besonders Polyethylenglykole (Polyethylenoxide), Polypropylenglykole (Polypropylenoxide), sowie Kopolymere aus Ethylenglykol (Ethylenoxid) und Propylenglykol (Propylenoxid) insbesondere gewisse Derivate dieser.
Wie bereits erwähnt, ist die Anbindung von Polyalkylenoxiden an Proteine (z. B.: Patent US 4, 179,337 (1979): "Non-immunogenic Polypeptides"), speziell auch an Hämoglobine, namentlich auch an künstliche Sauerstoffträger auf der Basis modifizierter Hämoglobine, bekannt (Patentschriften US 5,478,805 (1995): "Fractionation of Polyalkylene Oxide-Conjugated Hemoglobin Solution", US 5,386,014 (1995): "Chemically Modified Hemoglobin as an Effective, Stable, Nonimmunogenic Red Blood Cell Substitute", EP-A 0 206 448 (1986): "Hemoglobin Combined with a Poly (Alkylene Oxide)", EP-A 0 607 029 (1982): "Oxygen Carrier"). Der Inhalt dieser Schriften ist daher vorliegend inkorporiert. Die Anknüpfung von Polyalkylenoxiden an künstliche Sauerstoffträger auf der Basis modifizierter Hämoglobine wird nach der bekannten Literatur nur an unvernetztem Hämoglobin vorgenommen.

So beschreibt EPA 0 067 029 die Anknüpfung von Polyalkylenglykol, z. B. Polyethylen-/propylenglykol oder Copolymeren von Ethylenoxid/Propylenoxid oder einem Ether der genannten Glykole mit einem C₁- C₁₆-Alkohol, einem Ester aus den genannten Glykolen mit einer C₂-C₁₈-Carbonsäure (vorzugsweise Butyl/Monostearylester) und einem Amid aus Glykol und einem C₁-C₁₈-Amin (z. B. Propyl-Stearylamin). Als Verknüpfer werden z. B. N-Hydroxysuccinimid, N-Hydroxyphthalimide, p-Nitrophenol, Pentachlorphenol erwähnt. Analog können reaktive Derivate der genannten Polyalkylenglykol-Produkte eingesetzt werden. Das Molekulargewicht der Polymeren (z. B. Polyether) kann 300-20000, insbesondere 750-10000 sein. Molare Mengenverhältnisse und Reaktionstemperatur richten sich nach den jeweiligen beschriebenen und bekannten Bedingungen (vgl. Beispiele), z. B. 1-40-facher Überschuss PolyaLkylenoxid/Derivat pH von 7 bis 10.
Auch kann Hämoglobin mit Effektoren hier - wie erwähnt - verbunden sein, wie z. B. Pyridoxal-5'-phosphat oder Pyridoxal-5'-sulfat.
Die EPA 0 206 448 beschreibt ebenfalls die Anknüpfung von Polyalkylenoxiden wie oebengenannt, welche eine Aminofunktion aufweisen und somit über eine Amidbindung mit Hämoglobin verbunden sind. Das Molekül weist die Formel -CH₂-O-(CH₂)-CONHHb (n > 1. Insbesondere 1-10) auf. In den Beispielen 1-5 wird die Verknüpfung z. B. mit derivatisiertem Polyethylenglykol, beschrieben, z. B. auch bei Verwendung von Pyridoxal-5'-phosphat-Hämoglobin.

Die US-Patente 5 312 808 und 5 478 805 beschreiben die Herstellung von Hämoglobin-enthaltenden Lösungen mit Polyalkylenoxid-konjugiertem Hämoglobin mit einem Molekulargewicht größer 85000 Dalton, vgl. insbesondere die Beispiele 1-4, worin die Reaktionsbedingungen angegeben sind.
Gemäß der DE-OS 30 26 398 wird (nicht aktiviertes Polyethylenglykol mit der 2- bis 5-fachen molaren Menge Bromcyan (ph-Wert 9-10) umgesetzt. Das restliche Bromcyan wird durch Gelfiltration, Dialyse etc. aus dem Reaktionsgemisch entfernt und das Produkt wird dann mit einer erforderlichen, z. B. 0,1- bis 0,002-fachen, Menge Hämoglobin (pH 7 bis 9) in wässriger Lösung umgesetzt. Alternativ wird Polyethylenglykol in Benzol gegeben und mit der 2- bis 5-fachen molaren Menge Cyansäurechlorid umgesetzt. Das Reaktionsprodukt, Polyethylenglykol-4,5-dichlors-triazin, wird mit der gewünschten Menge, z. B. 1 bis 0,002 mol, Hämoglobin in einer Pufferlösung umgesetzt.

Die vorstehend erläuterten Methoden lassen sich auch im Fall der anderen genannten Polymeren sowie auch auf Myoglobin anwenden.

Das Hämoglobin,Myoglobin kann wie erwähnt nativ ( in aktivierter oder inaktivierter Form) vorliegen. Positiv ist hierbei die sigmoide Bindungscharakteristik des Hämoglobins, da hierdurch der Sauerstoff aus der Luft in großen Mengen gebunden und somit gespeichert und zugleich effektiv wieder diffusiv, gemäß dem FICKschen Gesetz, an die vitalen Zellschichten der Epidermis abgegeben werden kann. Wird zusätzlich Myoglobin eingesetzt, so hat dies den weiteren Vorteil, dass dessen Molekulargewicht viermal kleiner ist als das des Hämoglobins, so dass ein noch tieferes Eindringen des Sauerstofftransportmoleküls in die Haut möglich ist.

Mit Hilfe von Effektoren der Sauerstoffbindung kann die erfindungsgemässe Zubereitung noch verbessert werden. Hierdurch lassen sich die oben erläuterten Charakteristika der Sauerstoffbindung (p50 und n50) für den gewünschten Zweck optimieren. Es ist daher bevorzugt, dass bei der Herstellung der Lösung des Sauerstoffträgers , der nativ oder wie beschrieben modifiziert sein kann, bekannte natürliche Effektoren, wie z.B. 2,3.Diphosphoglycerat oder künstliche Effektoren wie Inositolhexaphosphat oder Mellitsäure, in 1-3facher, insbesondere etwa äquivalenten Mengen, bezogen auf Hämoglobin oder Hämoglobin/Myoglobin, hinzugesetzt werden. (Bamikol et al. Funkt.Biol.Med. 2 (1983) 245-249). Natürliche Effektoren, welche nicht chemisch reagieren, d.h. konformativ an das Hämoglobin/Myoglobin gebunden sind ,finden sich beispielsweise in "Lehninger et al., "Prinzipien der Biochemie", Spektrum Verlag, 1994 beschrieben.
Darüberhinaus kann das oben beschriebene Hämoglobin oder Myoglobin auch, bevorzugt auch zusätzlich zu den obengenannten Effektoren, chemisch modifiziert sein mit Effektoren, welche kovalent an das Hämoglobin gebunden werden. Hierzu gehören beispielsweise Pyridoxal-5-phosphat. Die Herstellung solcher modifizierter Hämoglobine ist in Kothe et al. Surgery 161 (1985) 563-569 beschrieben. Alternativ kann auch 2-Nor-2-Formyl-Pyridoxal-5-phosphat (van der Plas et al; Transfusion 27 (1985)425-430) als Effektor verwendet werden. Kovalent bindende Effektoren können sowohl für Hämoglobin als auch für Myoglobin verwendet werden.
Ganz besonders bevorzugt wird für die erfindungsgemäße Zubereitung ein wie folgt hergestellter Sauerstoff-Träger eingesetzt:
Monomeres Hämoglobin/Myoglobin, insbesondere desoxygeniert, wird in wässrigen Elektrolyten (z. B. NaHCO₃, NaCl, Na-Lactat oder Mischungen hiervon) mit einem Überschuss an Polyalkylenoxid, beispielsweise Polyethylen/propylenglykol (-oxid), Copolymerisate hiervon oder Derivate hiervon, insbesondere einem aktivierten Polyethylenglykol wie Methoxy-Polyethylenglykol-N-Hydroxysuccinimiddylpropionat (mPEG-SPA) mit dem gewünschten Molekulargewicht wie geschrieben, vemetzt. Der Überschuss an Reaktand kann auf bekannte Weise (Lysin) entfernt werden. Dabei kann vorzugsweise ein Effektor z. B. kovalent angeknüpft. sein, oder wie geschildert - konformativ wirkend der Lösung später zugesetzt werden. Ein wie oben beschrieben hergestelltes Hämoglobin/Myoglobin kann z. B. chromatographisch (z. B. durch präparative Volumenausschluss-Chromatographie) durch Zentrifugation, Filtration oder Ultrafiltration gereinigt und nachfolgend in der beschriebenen Weise der erfindungsgemässen Emulsion weiterverarbeitet werden. Gegebenenfalls erfolgt dann Stabilisation durch Karbonylierung.

Alternativ wird nicht modifiziertes natives Hämoglobin und Myoglobin eingesetzt, welches insbesondere bevorzugt durch Karbonylierung gegen Oxidation geschützt sein kann, wobei die Sauerstoffträgerlösung einen nicht chemisch reaktiven Effektor, wie erwähnt insbesondere 2,3-Diphophoglycerat aufweist, in einer 1 bis 3 fachen, vorzugsweise äquivalenten Menge bezüglich des Hämoglobins/Hämoglobins/Myoglobins. Ferner kann zusätzlich oder alternativ auch mit Pyridoxal-Effektoren chemisch modifiziertes Hämoglobin wie von Kothe und van der Plas beschrieben eingesetzt werden. Dazu wird Hämoglobin mit den entsprechenden genannten Effektoren umgesetzt, gegebenenfalls karbonyliert.

Ganz bevorzugt wird erfindungsgemäss desoxygeniertes, gegebenenfalls karbonyliertes nicht modifiziertes Human- oder inbesondere Schweinehämoglobin und entsprechendes desoxygeniertes, nicht modifiziertes Myoglobin vom Hund, oder Schaf, Pferd eingesetzt.

Mit Hilfe der erfindungsgemäßen Zubereitung werden überraschenderweise die Eigenschaften der molekular-dispersen Hämoglobine für eine möglichst effektive perkutane Sauerstoffdiffusion so optimiert, dass eine günstige Sauerstoffversorgung der Haut extern möglich wird.

Solche, mit Hilfe des Mechanismus der erleichterten Diffusion, Sauerstoff-transportierende Hämoglobin/Myoglobin-haltigen Zubereitungen sind nicht nur medizinisch von großem Interesse, sondern auch aus Sicht der Kosmetik. Denn der Alterungsprozeß der Haut ist auch entscheidend durch eine verringerte Sauerstoff-Verfügbarkeit für die vitale, hochaktive Zellschicht der Epidermis mitbedingt.Somit ist eine kosmetische Behandlung mit dem erfindungsgemäßen Mittel zugleich eine medizinische und umgekehrt.

Daher eignen sich die erfindungsgemäßen Hämoglobin- oder Hämoglobin/Myoglobinhaltigen Zubereitungen insbesondere auch als Mittel zur Behandlung von insbesondere altersbedingten Sauerstoffmangelzuständen der Haut, neben der Behandlung der Sauerstoffmangelzustände allgemein oder durch dauerdegenerative und/oder strahlungs- sowie thermisch bedingte Hautveränderungen - hierzu zählt auch die Narbenbildung - sowohl präventiv als auch therapeutisch, insbesondere auch als gleichzeitige Co-Therapie mit intravasaler Anwendung künstlicher Sauerstoffträger.
Wird ein CO-stabilisiertes Produkt verwendet, kann , nach dem Einbringen in die Haut das Hämoglobin oder das Hämoglobin und Myoglobin mit reinem Sauerstoff durch kurzzeitiges, etwa halbstündiges Begasen von außen, jedoch ohne Überdruck, als Sauerstoff-Binder reaktiviert werden, d. h. der Stabilisator wird entfernt. Von da ab transportiert der gelöste künstliche Sauersoffträger diffusiv verstärkt Sauerstoff auch aus der Luft, welche nur rund 20 % Volumenprozent Sauerstoff enthält. Es wird somit bevorzugt, dass das eingesetzte Hämoglobin oder Myoglobin vor der eigentlichen Anwendung gegen Oxidation geschützt, also stabilisiert ist.
Alternativ kann das Sauerstoff-transportierende Hämoglobin/Myoglobin auch ohne Stabilisation (Oxidationsschutz) angewendet werden. Obgleich ein solches Präparat nicht so lange haltbar ist wie ein CO-stabilisiertes, hat es den Vorteil, direkt ohne vorherige Aktivierung mit reinem Sauerstoff wirken zu können.

Das nicht stabilisierte Produkt eignet sich daher eher für die häusliche Anwendung, während das stabilisierte Produkt insbesondere für die ambulante oder stationäre Ersttherapie zum Einsatz kommen kann.

Vorteilhaft ist die Anwendung der erfindungsgemäßen Emulsion insbesondere auch in Kombination mit einer intravasalen Sauerstofftherapie, wobei als Sauerstoff-Träger die genannten Hämoglobine z.B. in Form einer isotonischen Kochsalzlösung als Infusion verabreicht werden können.

Darüberhinaus hat es sich als vorteilhaft erwiesen, wenn die erfindungsgemäße Emulsion, ggf. auch in Kombination mit einer intravasalen Therapie wie oben beschrieben, zusammen, das heißt vor oder nach der Anwendung, mit einem Gel erfolgt, welches ebensfalls die vorstehend genannten Sauerstoff-Träger enthält. Dieses Gel enthält eine Lösung des Sauerstoffträgers, insbesondere Hämoglobin oder Hämoglobin und Myoglobin insbesondere in einer Wasser und ggf. Salze enthaltenden Lösung in einer Zubereitung gelartiger Konsistenz molekular-dispers eingearbeitet ist, wozu eine Gel-bildende Substanz (0,1-20, vorzugsweise 0,1-8%) vorhanden ist. Insbesondere ist die gesamte Lösung in ein Gel eingearbeitet, wie anorganische Gele (Bentonit, Kieselsäure) und auf organischer Grundlage, wie Polyacrylsäure, Gummiarabicum, Pectinalginate, Methylcellulose, Hydroethylcellulose, Stärke sowie Carboxymethylcellulose. Vorzugsweise ist das Gel ein Hydrogel, ausgewählt aus anionischen Polyacrylaten, insbesondere Carbopot® der verschiedenen Typen wie Carbopol 940 oder 940 P. Das Gel ist fettfrei.
Als Salze können natürliche Elektrolytkomponenten der Lösung des Hämoglobins/Myoglobins wie NaCl, KCI und NaHCO₃, insbesondere in folgenden physiologischen Mengen vorliegen (in mM): NaCl 125; KCl 4,5; NaHCO₃ 2,0.
Das verwendete Gel enthält auch Konservierungsstoffe wie Dibromhexamidin, Dihydracetatsäure, 4-Hydroxybenzoesäure, Benzoesäure, Propionsäure, Salicylsäure, Sorbinsäure, Formaldehyd, Paraformaldehyd, O-Phenylphenol, anorganische Sulfite und Bisulfite, Natriumjodat, Chlorobutanol und Ameisensäure; in Fällen von Säuren kommen auch deren Ester und Salze in Frage. Die Mittel können in Mengen von 0,02-0,25 oder wie unten geschildert vorhanden sein.
Bevorzugt werden Konservierungsmittel ausgewählt aus Methyl-4-Hydroxy-Benzoat und Propyl-4-Hydroxy-Benzoat, z.B. 0,02-0,25, insbesondere 0,05-0,2, ganz besonders 0,09-0,17 %. Femer können bevorzugt Feuchthaltemittel wie Na-Lactat,Glycerol, Propylenglycol, Propandiol, Sorbit, PCA (Pyrrolidoncarbonsäure), in einer Menge von 5-15% enthalten sein. Besonders bevorzugt sind Methyl-4-Hydroxy-Benzoat und Propyl-4-Hydroxy-Benzoat (0,02-0,25%) als Konservierungsmittel und Glycerol, Propylenglycol und Sorbit als Feuchthaltemittel, insbesondere jeweils Mischungen hiervon, z.B. 1:1 betreffend Konservierungsmittel und 1:1:1 bezüglich Feuchthaltemittel. Ein besonders bevorzugtes erfindungsgemässes Präparat gelartiger Konsistenz umfasst Hämoglobin oder Hämoglobin/Myoglobin (ca. 0,1-30%, insbesondere 0,1-20%, bezogen auf die Gesamtmasse), 5-15 Gew.-% Feuchthaltemittel, 0,15-0,25 % Konservierungsmittel, und 0,1 bis 20 %, insbesondere 0,1 bis 8 % Carbopol®. Ganz besonders bevorzugt sind Präparate auf Basis von 1-5% Carbopol®, 0,02-0,08 Propyl-4-Hydroxybenzoat, sowie 0,07-0,15 Masseteile Methyl-4-Hydroxybenzoat, 8-12% Glycerol, Propylenglycol und/oder Sorbit (1:1 oder 1:1:1). Bevorzugt liegt Hämoglobin oder Hämoglobin und Myoglobin in einer Menge von 2-8% vor. Die angegebenen Mengen sind jeweils Gewichtsprozent und die Mischungsverhältnisse von Hämoglobin/Myoglobin entsprechend den vorstehend für die Emulsion genannten.

Das Hämoglobin im Gel ist analog wie in der Emulsion, also nativ, und/oder mit Polyalkylenoxid vemetzt, und/oder mit Konformativ- und/oder Kovalent-Effektoren versetzt und/oder desoxygeniert und kann wie beschrieben und unten unter Schritt **I** erläutert auch hergestellt werden. Das Gel wird hergestellt, indem eine vorstehend beschriebene Sauerstoff-Träger-Lösung in eine wäßrige Lösung eingearbeitet wird, die das Gel und die Zusatzstoffe enthält. Eine derartige Lösung ist nachfolgend beschrieben unter **Schritt II**, wobei statt der Ölkomponente und des Emulgators das Gel inkorporiert ist. Das Gel kann dabei hergestellt werden, indem 0,1-15g, insbesondere 1-10g der gelbildenden Substanz, insbesondere des Hydrogels, wie z.B. Carbopol®, z.B.1-5g/Liter Wasser oder aqua conservans zugesetzt werden. Dieser Mischung werden dann analog die Zusatzstoffe beigefügt und schließlich die wie oben und insbesondere nachfolgend unter **Schritt III** beschrieben, die Sauerstoff-Trägertösung hinzugefügt.

Das Gel und die Emulsion können unmittelbar aufeinander folgend in beliebiger Reihenfolge und auch in einem zeitlichen Abstand von 1 bis 12 Stunden angewendet werden.
Hierzu werden beide Zubereitungen wie beschrieben getrennt hergestellt und konfektioniert und können z.B. in einer Gesamtverpackung bereitgestellt werden für die Anwendung. Hierbei ist dann das jeweilige Produkt namentlich und ggf zusätzlich z.B. durch eine andersartige Farbe gekennzeichnet. Beispielsweise kann das Gel blau oder grün, die Creme/Lotion rot oder gelb markiert sein.

Die Erfindung wird nun weiter erläutert anhand einer allgemeinen Verfahrensvorschrift zur Herstellung der Emulsion sowie den nachfolgenden Anwendungsbeispielen. Die Herstellung erfolgt in drei Schritten (**I, II** und **III**).

### Schritt I:

Es wird eine konzentrierte Lösung des Hämoglobins oder des Hämoglobins und Myoglobins, insbesondere des Human-, Schweine- oder Rinderhämoglobins bzw. Rind/Schaf/Pferd-Myoglobins, welche nicht modifiziert, oder auch bevorzugt chemisch modifiziert oder mit einem nicht chemisch reaktiven Effektor versehen, wie auch pegyliert sind, in einem Konzentrationsbereich zwischen 150 und 450, bevorzugt zwischen 300 und 400 g/l hergestellt. Für die ambulante Anwendung werden Hämoglobin und gegebenenfalls Myoglobin durch Schütteln mit reinem Kohlenmonoxid (CO) vollständig karbonyliert. Die Lösung bleibt hierbei in oben genanntem Konzentrationsbereich. Den Hämoglobinen kann, wie erwähnt, bis zu 50 Gewichtsprozent Myoglobin beigemischt sein.
Die Lösung enthält auch zwischen 15 und 80, bevorzugt zwischen 40 und 60mM NaHCO₃ sowie zwischen 80 und 250, bevorzugt zwischen 100 und 200 mM NaCl. Im Fall der Herstellung einer Zubereitung für die häusliche und selbständige Nach-Therapie sowie für die häuslichen kosmetische Anwendung unterbleibt die Karbonylierung der Hämoglobin/Myoglobin-Lösung.

### Schritt II:

Man geht von Aqua conservans aus. Dieses kann aus der Apotheke bezogen oder gemäß NRF (Neues Rezept Formulatorium) S.6 selbst hergestellt werden, Zusammensetzung von aqua conservans siehe dort.
Zur eigenen Herstellung verwendet man gereinigtes Wasser und fügt die Konservierungsmittel, insbesondere 0,02 bis 0,08 Propyl-4-Hydroxybenzoat, bevorzugt jedoch 0,025 sowie 0,07 bis 0,15 Massenanteile Methyl-4-Hydroxybenzoat, bevorzugt jedoch 0,075 hinzu.
Als Feuchthaltemittel, zum Weichmachen der Haut, setzt man zwischen 5% und 15 % Masseanteile z.B. an Glycerol, Propylenglykol oder 70%ige Sorbitlösung gemäß DAB 9, bevorzugt 8 bis 12% zu. Alternativ kann man zwei oder drei der genannten Feuchthaltemittel in der angegebenen (Gesamt)Menge, bevorzugt zu gleichen Teilen, insbesondere mit einem Gesamtmasse-Anteil zwischen 8 und 12%, zusetzen.
Dieser Lösung setzt man einen geeigneten Emulgator in einem Gewichtsanteil von 2 bis 20, bevorzugt 2-10, insbesondere 2-5 oder 3-4,5% zu, ferner Fette/Öle zwischen 10 und 80, bevorzugt zwischen 20 und 60 Gewichts%. Fakultativ können noch alle oder einzelne der genannten Adjuvantien zugesetzt werden: Erstens hyperämisierende Substanz zwischen 0 und 3, bevorzugt zwischen 0 und 1,5 Gewichts%; zweitens ätherische Öle zur Förderung der Durchblutung, zur anti-infektiösen Wirkung und zur Regeneration (Narbentherapie), alle in Gewichtsanteilen zwischen 0 und 13, insbesondere zwischen 2 und 10. Schließlich kann die Zubereitung Antioxidantien, wie oben beispielhaft genannt, enthalten, und zwar zwischen 0,0001 und 0,5, bevorzugt zwischen 0,01 und 0,08Gew.%.
Durch Einbringen in einen geeigneten Mischer wird schließlich die Emulsion hergestellt.

### Schritt III:

Die Emulsion (aus II) wird anschließend mit der Lösung (aus I) derart gemischt, daß ein Gewichtsanteil an Hämoglobin (und Myoglobin) zwischen 0,1, insbesondere 1-30, bevorzugt 1-20 oder 1-15 Gewichtsprozent besteht, insbesondere zwischen 2 und 8. Die Emulsion kann noch einmal in einer Mischmaschine bearbeitet werden. Ein hoher Wassergehalt der Emulsion aus Schritt III führt zu einer Lotion, ein geringerer zu einer Creme. Lotion und Creme haben qualitativ die gleiche Zusammensetzung.

### Anwendungsbeispiel 1

### Erfindungsgemäße Herstellung einer kombinierten Emulsion in Form einer Lotion

### I Herstellung der Emulsionsgrundlage

Die Emulsion enthält auf 100 Masseteile:
(vergleiche auch DAC. - NRF S. 25)

| | Masseteile |
|---|---|
| Sorbitanmonostearat | 2,00 Teile |
| Macrogol-9-Stearat | 3,00 Teile |
| Glycerol 85 % | 5,00 Teile |
| Mittelkettige Triglyceride | 5,00 Teile |
| Wasserfreie Citronensäure | 0,07 Teile |
| Kaliumsorbat | 0,14 Teile |
| Gereinigtes Wasser | 84,79 Teile |

Die Zubereitung geschieht folgendermaßen:

In einer tarierten 1000-ml-Gewindeflasche aus Glas werden Sorbitanmonostearat, Macrogol-9-Stearat, Glycerol 85 % und Mittelkettige Triglyceride auf dem Wasserbad erwärmt, bis die festen Bestandteile geschmolzen sind. Wasserfreie Citronensäure und Kaliumsorbat werden hinzugefügt. Mit auf mindestens 60 °C erwärmtem Gereinigtem Wasser wird der Ansatz zu 500,0 g ergänzt. Die Flasche wird verschlossen, kräftig geschüttelt und unter weiterem gelegentlichem Schütteln bis zum Erkalten stehengelassen.

### II Herstellung der Sauerstoff-Träger-haltigen Emulsion

84,0 g der unter I hergestellten Emulsionsgrundlage werden zu 16,0 g einer 30 %igen (Gewicht) Schweinehämoglobinlösung gegeben; die Hämoglobinlösung enthält 50 mM Natriumbikarnonat und 150 mM Natriumchlorid. Durch Schütteln wird die Hämoglobinlösung in die Emulsionsgrundlage eingearbeitet.

### Anwendungsbeispiel 2

### Erfindungsgemäße Herstellung einer Öl-Emulsion als Creme

In ein Rührwerk-Gefäß werden eingebracht:

| Öl von | |
|---|---|
| Aprikosenkernen | 5,00 g |
| Weizenkeimen | 5,00 g |
| Aloe vera | 5,00 g |
| Macadamia | 5,00 g |
| Schwarckümmel | 5,00 g |
| Sheabutter | 10,00 g |
| Aqua conservans | 35,00 g |
| (DAC. NRF S. 6.) | |

| | |
|---|---|
| Unguentum emulsificans (DAB 1997) | 30,00 g |
| D-∝-Tocopherol | 0,05 g |

Duftstoffe: natürliche ätherische Öle

| | |
|---|---|
| Neroli | 1 Tropfen |
| Geranie | 1 Tropfen |
| Mandarine | 1 Tropfen |

Die aufgeführten Bestandteile werden in einem Arbeitsvorgang unter Zuhilfenahme eines mechanischen Rührwerkes bei 1000 Umdrehungen pro Minute eine Minute lang emulgiert.

### Anwendungsbeispiel 3

### Herstellung eines Gels

1,5 g Methyl-4-Hydroxy-Benzoat und 0,5 g Propyl-4-Hydroxy-Benzoat wurden in bidestilliertem Wasser gelöst und mit diesem auf 1 l aufgefüllt (: "DAC").
5,0 g Carbopol® 940 werden mit 45 ml Glycerol und 45 ml 1,2 - Propandiol angerührt, der Mischung dann 850 ml DAC hinzugefügt und ferner 350 ml bidestilliertes Wasser sowie 38 ml 1 M NaOH zur Gel-Bildung. In das Gel ließen sich 280 ml einer Schweinehämoglobin-Lösung der Konzentration 280 g/l homogen einmischen. Das Hämoglobin war vorher zu 99% mit Kohlenmonoxid ligandiert worden.
Wie erwähnt, können Creme/Lotion und Gel auf die genannte Weise hergestellt werden und dann zusammen konfektioniert werden,z.B. in einer Gesamtverpackung, so dass bei der Anwendung beide Darreichungsformen gleichzeitig zur Verfügung stehen. Dabei sind die unterschiedlichen Produkte wie erwähnt gekennzeichnet und können auch jeweils verschieden markiert sein. Insbesondere können auch Angaben über die zeitliche Abfolge der Anwendung angebracht sein.

## Patentansprüche

1. Extern applizierbare Zubereitung, enthaltend einen Sauerstoffträger, ausgewählt aus Hämoglobin oder Hämoglobin und Myoglobin, **dadurch gekennzeichnet, dass** insgesamt 0,1-30% Hämoglobin oder Hämoglobin und davon 0,1-50-% Myoglobin, bezogen auf die Hämoglobin-Menge der Sauerstoffträger in einer lipoiden Emulsion molekular-dispers eingearbeitet ist.

2. Zubereitung nach Anspruch 1, **dadurch gekennzeichnet, dass** das Hämoglobin oder Myoglobin und Hämoglobin in eine Öl-in-Wasser-Emulsion eingearbeitet ist.

3. Zubereitung nach einem der Ansprüche 1 oder 2, **dadurch gekennzeichnet, dass** neben dem Sauerstoff-Träger 20 bis 90% Wasser, 10 bis 80 % Ölkomponente, 2 bis 20% Emulgator, 5 bis 15% Feuchthaltemittel, 0,02 bis 0,25 % Konservierungsstoffe und 0 bis 20% Zusatzstoffe, ausgewählt aus Parfümsubstanzen, Substanzen zur regenerativen Narbentherapie, Durchblutung , Antioxidantien und/oder anti-infektiösen Substanzen enthalten sind.

4. Zubereitung nach Anspruch 3, **dadurch gekennzeichnet, dass** als Ölkomponente Öle mit ungesättigtem Teil und/oder Fette aus kurz-und mittelkettigen gesättigten verzweigten und/oder unverzweigten Fettsäuren oder Mischungen hiervon enthalten sind.

5. Zubereitung nach einem der Ansprüche 3 oder 4, **dadurch gekennzeichnet, dass** als Emulgator ein nichtionischer Emulgator mit einem HLB-Wert von 8-18 , insbesondere in reiner Form ,oder Mischungen hiervon enthalten sind.

6. Zubereitung nach einem der Ansprüche 1 bis 5, **dadurch gekennzeichnet, dass** das Hämoglobin oder Myoglobin und Hämoglobin insgesamt in einer Konzentration von 0,1 bis 20 %, bezogen auf die Gesamtmenge vorliegt.

7. Zubereitung nach einem der Ansprüche 1 bis 6, **dadurch gekennzeichnet, dass** als Hämoglobin Human-, Schweine- oder Rinderhämoglobin und als Myoglobin solches vom Pferd, Hund oder Schaf eingearbeitet ist.

8. Zubereitung nach einem der Ansprüche 1 bis 7, **dadurch gekennzeichnet, dass** als Hämoglobin oder Myoglobin und Hämoglobin natives, chemisch modifiziertes und/oder gegen Oxidation geschütztes Hämoglobin oder Myoglobin und Hämoglobin eingearbeitet ist.

9. Zubereitung nach Anspruch 7 oder 8, **dadurch gekennzeichnet, dass** das Hämoglobin oder Myoglobin und Hämoglobin mit einem Polyalkylenoxid kovalent verknüpft und/oder mit einem natürlichen und/oder künstlichen Effektor kovalent und/oder konformativ versehen ist.

10. Zubereitung gemäß einem der Ansprüche 1 bis 9, **dadurch gekennzeichnet, dass** sie als Lotion mit einem Anteil von 70-90 Gew.% Wasser und einem Anteil von 1-20 Gew. % Ölkomponente oder in Form einer Creme mit einem Anteil von 40-80 Gew. % Wasser und 5-50Gew.% Ölkomponente vorliegt und die übrigen Bestandteile in den angegebenen Mengen vorhanden sind.

11. Verfahren zur Herstellung einer Sauerstoff-Träger-haltigen Zubeitung gemäß einem der Ansprüche 1 bis 10, **dadurch gekennzeichnet, dass** man in 20 bis 90% Wasser, 10 bis 80% einer oder mehreren Ölkomponenten zusammen mit 2-5% Emulgator unter Rühren hinzufügt, sodann die Zusatzstoffe, sofern vorhanden unter Rühren hinzufügt und anschließend den Sauerstoff-Träger in wässriger Lösung hinzufügt.

12. Verfahren zur Herstellung einer Sauerstoff Träger-haltigen Zubereitung zur externen Anwendung, **dadurch gekennzeichnet, dass** man eine Zubereitung gemäß einem der Ansprüche 1 bis 12, sowie ein Gel, enthaltend 5 bis 15 % Feuchthaltemittel, 0,02 bis 0,25 % Konservierungsmittel, eine Gel-bildende Substanz in einer Menge von 0,1-20 % sowie insgesamt 0,1-30% Hämoglobin oder Hämoglobin mit bis zu 0,1-50% Myoglobin , bezogen auf die Gesamtmenge jeweils einzeln herstellt und dann zusammen konfektioniert zur gemeinsamen Verabreichung.

13. Extern applizierbare Kombinationszubereitung, hergestellt nach dem Verfahren gemäß Anspruch 12, umfassend eine Sauerstoff-Träger-haltige Zubereitung in Form einer Emulsion und in Form eines Gels.

14. Verwendung einer Zubereitung gemäss einem der Ansprüche 1 bis 10, 13 oder hergestellt gemäss einem der Ansprüche 11 oder 12 zur Herstellung eines Mittels zur externen Behandlung/ Prävention von Sauerstoffmangelzuständen der Haut.

15. Verwendung gemäss Anspruch 14, **dadurch gekennzeichnet, dass** der Sauerstoffmangel durch degenerative und/oder strahlungs- sowie thermischbedingte Hautveränderungen hervorgerufen ist.

16. Verwendung einer Zubereitung gemäss einem der Ansprüche 1 bis 10,13 oder hergestellt gemäss einem der Ansprüche 11 oder 12 zur Herstellung eines Mittels zur Behandlung/ Prävention von altersbedingten Sauerstoffmangelzuständen der Haut.

17. Verwendung gemäß einem der Ansprüche 14 bis 16, **dadurch gekennzeichnet, dass** eine durch Karbonylierung stabilisierte und mittels Begasung der Haut mit reinem Sauerstoff aktivierte Zubereitung oder eine nicht stabilisierte Zubereitung für die häusliche Therapie eingesetzt wird.

18. Verwendung gemäß einem der Ansprüche 14 bis 17, **dadurch gekennzeichnet** das zusätzlich eine intravasale Sauerstofftherapie mit einem Hämoglobin-Sauerstoffträger erfolgt.

19. Verwendung einer Zubereitung gemäß einem der Ansprüche 1-10 als Kosmetikum für einen frischen Teint und natürliches Aussehen.

## Claims

1. Preparation for topical application containing an oxygen carrier selected from haemoglobin or haemoglobin and myoglobin, **characterized in that** a total, of 0.1-30% of haemoglobin or haemoglobin and 0.1-50% of myoglobin, based on the amount of haemoglobin in the oxygen carriers, is incorporated in a lipoid emulsion as a molecular dispersion.

2. Preparation according to Claim 1, **characterized in that** haemoglobin or myoglobin and haemoglobin is incorporated in an oil-in-water emulsion.

3. Preparation according to Claim 1 or 2, **characterized in that**, in addition to the oxygen carrier, it contains 20 to 90% of water, 10 to 80% of oil component, 2 to 20% of emulsifier, 5 to 15% of humectant, 0.02 to 0.25% of preservatives and 0 to 20% of additives selected from perfume substances, substances for regenerative scar therapy or circulation, antioxidants and/or anti-infective substances.

4. Preparation according to Claim 3, **characterized in that** the oil component it contains consists of partially unsaturated oils and/or of fats made up of short-chain and medium-chain, saturated, branched and/or unbranched fatty acids or mixtures thereof.

5. Preparation according to Claim 3 or 4, **characterized in that** the emulsifier it contains consists of a nonionic emulsifier with an HLB of 8-18, especially in the pure form, or mixtures thereof.

6. '. Preparation according to one of Claims 1 to 5, **characterized in that** the haemoglobin or myoglobin and haemoglobin is present in a total concentration of 0.1 to 20%, based on the total amount.

7. Preparation according to one of Claims 1 to 6, **characterized in that** the incorporated haemoglobin is human, porcine or bovine haemoglobin and the incorporated myoglobin is equine, canine or ovine myoglobin.

8. Preparation according to one of Claims 1 to 7, **characterized in that** the incorporated haemoglobin or incorporated myoglobin and haemoglobin is native, chemically modified and/or oxidation-protected haemoglobin or myoglobin and haemoglobin.

9. Preparation according to Claim 7 or 8, **characterized in that** the haemoglobin or myoglobin and haemoglobin is covalently coupled with a polyalkylene oxide and/or has a natural and/or artifical effector covalently and/or conformationally bonded to it.

10. Preparation according to one of Claims 1 to 9, **characterized in that** it takes the form of a lotion containing 70-90 wt.% of water and 1-20 wt.% of oil component, or a cream containing 40-80 wt.% of water and 5-50 wt.% of oil component, and the remaining constituents are present in the indicated amounts.

11. Process for the manufacture of a preparation containing an oxygen carrier according to one of Claims 1 to 10, **characterized in that** 10 to 80% of one or more oil components together with 2-5% of emulsifier is introduced into 20 to 90% of water, with stirring, and any additives are then introduced, with stirring, followed by an aqueous solution of the oxygen carrier.

12. Process for the manufacture of a preparation containing an oxygen carrier for topical use, **characterized in that** a preparation according to one of Claims 1 to 12, and a gel containing 5 to 15% of humectant, 0.02 to 0.25% of preservative, 0.1-20% of a gel-forming substance and a total of 0.1-30% of haemoglobin or haemoglobin with up to 0.1-50% of myoglobin, based on the total amount, are each manufactured individually and then combined for common application.

13. Combination preparation for topical application manufactured by the process according to Claim 12, comprising a preparation containing an oxygen carrier in the form of an emulsion and in the form of a gel.

14. Use of a preparation according to one of Claims 1 to 10 and 13, or manufactured according to Claim 11 or 12, for the manufacture of an agent for the topical treatment/prevention of oxygen deficiency states of the skin.

15. Use according to Claim 14, **characterized in that** the oxygen deficiency has been caused by degenerative skin changes and/or skin changes due to radiation and heat.

16. Use of a preparation according to one of Claims 1 to 10 and 13, or manufactured according to Claim 11 or 12, for the manufacture of an agent for the treatment/ prevention of oxygen deficiency states of the skin due to ageing.

17. Use according to one of Claims 14 to 16, **characterized in that** a preparation stabilized by carbonylation and activated by treatment of the skin with pure oxygen gas, or a non-stabilized preparation for home therapy, is used.

18. Use according to one of Claims 14 to 17, **characterized in that** an intravascular oxygen therapy with a haemoglobin oxygen carrier is additionally performed.

19. Use of a preparation according to one of Claims 1-10 as a cosmetic for a fresh complexion and natural appearance.

## Revendications

1. Préparation applicable par application externe contenant un support d'oxygène choisi parmi l'hémoglobine ou l'hémoglobine et la myoglobine, **caractérisée en ce que**, au total, 0,1-30 % d'hémoglobine ou d'hémoglobine et 0,1-50 % de myoglobine, par rapport à la quantité d'hémoglobine des supports d'oxygène sont incorporés sous forme de dispersion moléculaire dans une émulsion lipoïde.

2. Préparation selon la revendication 1 **caractérisée en ce que** l'hémoglobine ou la myoglobine et l'hémoglobine sont incorporées dans une solution huile-dans-eau.

3. Préparation selon l'une des revendications 1 et 2 **caractérisée en ce que**, outre le support d'oxygène, 20 à 90 % d'eau, 10 à 80 % de composant huileux, 2 à 20 % d'émulsifiant, 5 à 15 % d'agent de maintien de l'humidité, 0,02 à 0,25 % de conservateurs et 0 à 20 % d'additifs, choisis parmi les substances de type parfum, les substances pour la thérapie régénérative des cicatrices, la circulation, les antioxydants et/ou les substances anti-infectieuses sont contenus.

4. Préparation selon la revendication 3 **caractérisée en ce que**, comme composant huileux, des huiles ayant une partie insaturée et/ou des graisses constituées par des acides gras saturés à chaîne courte et moyenne, ramifiés et/ou non ramifiés, ou des mélanges de ceux-ci, sont contenues.

5. Préparation selon l'une des revendications 3 et 4 **caractérisée en ce que**, comme émulsifiant, un émulsifiant non ionique ayant une valeur HLB de 8-18, en particulier sous forme pure, ou des mélanges de ceux-ci sont contenus.

6. Préparation selon l'une des revendications 1 à 5 **caractérisée en ce que** l'hémoglobine ou la myoglobine et l'hémoglobine sont présentes au total en une concentration de 0,1 à 20 % par rapport à la quantité totale.

7. Préparation selon l'une des revendications 1 à 6 **caractérisée en ce que** de l'hémoglobine humaine,porcine ou bovine est incorporée comme hémoglobine et de la myoglobine de cheval, de chien ou de mouton est incorporée comme myoglobine.

8. Préparation selon l'une des revendications 1 à 7 **caractérisée en ce que**, comme hémoglobine ou comme myoglobine et hémoglobine, de l'hémoglobine ou de la myoglobine et de l'hémoglobine native, chimiquement modifiée et/ou protégée contre l'oxydation est incorporée.

9. Préparation selon la revendication 7 ou 8 **caractérisée en ce que** l'hémoglobine ou la myoglobine et l'hémoglobine est liée de manière covalente à un poly(oxyde d'alkylène) et/ou est munie de manière covalente et/ou par conformation d'un effecteur naturel et/ou artificiel.

10. Préparation selon l'une des revendications 1 à 9 **caractérisée en ce qu'**elle est sous forme d'une lotion ayant une fraction de 70-90 % en masse d'eau et une fraction de 1-20 % en masse de composant huileux ou sous forme d'une crème ayant une fraction de 40-80 % en masse d'eau et 5-50 % en masse de composant huileux, et les autres constituants sont présents en les quantités indiquées.

11. Procédé de production d'une préparation contenant un support d'oxygène selon l'une des revendications 1 à 10 **caractérisé en ce que** l'on ajoute sous agitation à 20 à 90 % d'eau, 10 à 80 % d'un ou plusieurs composants huileux. en même temps que 2-5 % d'émulsifiant, puis on ajoute sous agitation les additifs, s'ils sont présents, et ensuite on ajoute le support d'oxygène en solution aqueuse.

12. Procédé de production d'une préparation contenant un support d'oxygène pour l'application externe, **caractérisé en ce que** l'on produit individuellement dans chaque cas une préparation selon l'une des revendications 1 à 12, ainsi qu'un gel contenant 5 à 15 % d'agent de maintien de l'humidité, 0,02 à 0,25 % de conservateur, une substance gélifiante en une quantité de 0,1-20 % ainsi que, au total, 0,1-30 % d'hémoglobine ou d'hémoglobine avec jusqu'à 0,1-50 % de myoglobine, par rapport à la quantité totale, puis on les confectionne conjointement pour l'administration commune.

13. Préparation de type combinaison applicable par application externe produite par le procédé selon la revendication 12 comprenant une préparation contenant un support d'oxygène sous forme d'une émulsion et sous forme d'un gel.

14. Utilisation d'une préparation selon l'une des revendications 1 à 10, 13 ou produite selon l'une des revendications 11 et 12 pour la production d'un agent pour le traitement externe/la prévention d'états de manque d'oxygène de la peau.

15. Utilisation selon la revendication 14 **caractérisée en ce que** le manque d'oxygène est provoqué par des modifications cutanées dégénératives et/ou dues à un rayonnement ou à la chaleur.

16. Utilisation d'une préparation selon l'une des revendications 1 à 10, 13 ou produite selon l'une des revendications 11 et 12 pour la production d'un agent pour le traitement/la prévention d'états de manque d'oxygène de la peau dus à l'âge.

17. Utilisation selon l'une des revendications 14 à 16 **caractérisée en ce qu'**une préparation stabilisée par carbonylation et activée par traitement de la peau avec du gaz oxygène pur ou une préparation non stabilisée est utilisée pour la thérapie domestique.

18. Utilisation selon l'une des revendications 14 à 17 **caractérisée en ce qu'**une thérapie intravasculaire à l'oxygène a lieu en outre avec un support d'oxygène à l'hémoglobine.

19. Utilisation d'une préparation selon l'une des revendications 1 à 10 comme cosmétique pour une teinte fraîche et un aspect naturel.
